# EUROPEAN PATENT APPLICATION

(11) **EP 1 923 016 A1**
(43) Date of publication of application: **21.05.2008**
(21) Application number: 06782243.7
(22) Date of filing: 03.08.2006
(51) Int. Cl.: A61B 19/00

(54) **SURGICAL AUXILIARY TOOL FOR COVERING OF EXO-SEROUS MEMBRANE EXPOSED TUMOR AND METHOD OF PREVENTING DISSIPATION OF TUMOR CELL ATTRIBUTED TO INTRA-BODY CAVITY SURGICAL MANIPULATION THEREWITH**

(30) Priority: 11.08.2005 JP 2005232805
(71) Applicant: National University Corporation Kobe University, Kobe-shi, Hyogo 657-8501 (JP)
(72) Inventor: ICHIHARA, Takao, Kobe-shi, Hyogo 6578501 (JP); TAKADA, Moriatsu, Kobe-shi, Hyogo 6578501 (JP)
(74) Representative: Dossmann, Gérard
(86) International application number: PCT/JP2006/315383
(87) International publication number: WO 2007/018115

(57) **Abstract**

A convenient surgical auxiliary tool for covering of exo-serous membrane exposed tumor, capable of preventing any man-caused dissipation of tumor cells attributed to surgical manipulation in the digestive organs, etc. There is provided a surgical auxiliary tool comprising a pair of frame members having the respective free end portions rotatably coupled with each other; an accommodation bag having its rim of opening portion fixed to the coupled pair of frame members; and a locking part disposed at substantially the center of the frame members and capable of maintaining the closed state of the pair of frame members. By virtue of this construction, the intestine on the periphery of an area of infiltration of the serous membrane can be pinched by means of the pair of frame members having the respective free end portions rotatably coupled with each other. Simultaneously, the intestine on the periphery of an area of infiltration of the serous membrane can be covered by the accommodation bag having its rim of opening portion fixed to the frame members. Further, the surgical auxiliary tool can be taken out from the body cavity inside to the body cavity outside while surely maintaining the state of covering the intestine on the periphery of an area of infiltration of the serous membrane by means of the locking part capable of maintaining the closed state of the coupled pair of frame members.

## Description

### [Technical Field]

The present invention relates to a surgical auxiliary tool in a surgical operation of mainly digestive organs, specifically the surgical auxiliary tool used for performing an operation procedure after covering when growing cancer tissues infiltrated and exposed in an area of exo-serous membrane and for covering of exo-serous membrane exposed tumor for the purpose of preventing any dissemination and metastasis of tumor cells attributed to a surgical manipulation itself, and a method for preventing any man-caused dissipation of tumor cells attributed to the intra-body cavity surgical manipulation using the same.

### [Background Art]

In the surgical operation, an operator sometimes encounters the manipulation of an advanced cancer exposed out of the organ, and it has become a problem in terms of a medical standpoint that even when a primary focus has been removed, reoccurrence attributed to dissemination of cancer cells due to the surgical manipulation itself is observed. In such a case, in the conventional operation procedure, when the advanced cancer exposed out of the organ was found, the operation was often stopped because the metastasis had already been regarded to occur.

On the other hand, with progress in recent years, advanced cancer having the infiltration out of the organ has tended to be exsected. In such a case, when the cancer cells are removed, it is a real situation that only means of advancing the operation procedure by covering the periphery with gauze is available. Thus, it has been described that it is impossible to perform the operation in such a case in scopic surgery, and it has been anticipated to develop a covering device for preventing the dissemination and metastasis of cancer cells.

Also in routine operations, the dissemination during the operation due to the surgical manipulation becomes problematic, and it has been required in a surgical operation that the operation is performed without contacting with tumor.
Conventionally, in methods of removing an abnormal tissue from a digestive tract of a patient, the method has been known in which an endoscope is introduced in the digestive tract in the patient, the abnormal tissue is suctioned in a ligating tool at a distal end of the endoscope by applying suctioning force, a polyp composed of the abnormal tissue is formed by applying a ligating band to a base of the abnormal tissue in the ligating tool, and the polyp is removed from the peripheral tissue (Patent Document 1). However, in the case of advanced cancer where the abnormal tissue has been infiltrated out of the organ, it is difficult to apply this abnormal tissue removal method.

From these circumstances, a medical covering device is currently required which easily, safely and reliably covers a cancer tissue exposedportion in the removal of advanced cancer having infiltration out of the organ in the surgical field.

[Patent document 1] JPA1998-014930

### [Disclosure of the Invention]

### [Problems to be Solved by the Invention]

The surgical auxiliary tool for covering of exo-serous membrane exposed tumor according to the present invention has been made by focusing on the above circumstances, and is capable of easily, safely and reliably covering the exposed tumor even under the condition such as a surgical operation, particularly a scopic surgery where space for use of tools cannot be sufficiently assured. The present invention aims at providing a simple surgical auxiliary tool for covering of exo-serous membrane exposed tumor and capable of preventing any man-caused dissipation of tumor cells attributed to intra-body cavity surgical manipulation, and a method for preventing the dissipation of tumor cells attributed to the intra-body cavity surgical manipulation using the same.

### [Means to Solve the Objects]

The present inventors are doctors specializing in scopic surgery, have invented the surgical auxiliary tool for covering of exo-serous membrane exposed tumor according to the present invention through a wide range of clinical experience of scopic techniques, and have completed the present invention by making various prototypic products and improving them.

In order to achieve the above object, the surgical auxiliary tool for covering of an exo-serous membrane exposed tumor is composed of a pair of frame members having the respective free end portions rotatably coupled with each other; an accommodation bag having its rim of opening portion fixed to the coupled pair of frame members; and a locking part disposed at substantially the center of the frame members and capable of maintaining the closed state of the pair of frame members. Here, the pair of frame members is not necessarily, strictly speaking, the pair of frame members, and is used in a sense that they could have the same shape to an extent that the intestine can be pinched when they are overlayed using the coupled portion as a supporting point.

Accordingly, the intestine on the periphery of an area of infiltration of the serous membrane can be pinched by means of the pair of frame members having the respective free end portions rotatably coupled with each other. Simultaneously, the intestine on the periphery of the area of infiltration of the serous membrane can be covered by the accommodation bag having its rim of opening portion fixed to the frame members.

Further, the surgical auxiliary tool can be taken out from the body cavity inside to the body cavity outside while reliably maintaining the state of covering the intestine on the periphery of an area of infiltration of the serous membrane by means of the locking part capable of maintaining the closed state of the coupled pair of frame members. It is assuredly possible to cover the exposed tumor and simply prevent any man-caused dissipation of tumor cells attributed to the intra-body cavity surgical manipulation.

More specifically, in the above constitution, it is preferable that an entire shape of this frame member is a Y-shape, a U-shape or more notably, a horseshoe shape, since the Y-shape, the U-shape or the horseshoe shape can pinch the intestine with a given distance by its foot part toward the free end portion. It has also an advantage that the device can be easily inserted in and taken out from an incision site upon operation.

Particularly, the case of the Y-shape further has the advantage that it becomes possible to control an interval distance of pinching the intestine by controlling the position of the foot part of pinching the intestine. Since the foot part of the frame member has a bar shape, the intestine is pinched with a bar member. Thus, there is advantage that in the pinched state, a width of the intestine is pushed to extend, a constriction part is reduced and it is not necessary to exsect any extraneous parts upon exsecting.

It is also preferable that the surface of the frame member is covered with a covering member composed of a soft and flexuous resin. Covering the surface with the covering member composed of the flexible resin is for protecting so as not to injure the surface of the intestine when the frame members directly contact with the intestine to pinch it.

The respective free end portions of the frame member being rotatably coupled with each other is that the coupled portion of the free end portions and the covering member are integrated using the soft and flexuous resin, or that the coupled portion of the free end portion has a hinge mechanism of rotatably coupling the free end portions with a pin and the like.
When the coupled portion of the free end portions of the frame member and the covering member are integrated using the soft and flexuous resin, there is an advantage that a working step of covering the frame member surface with the flexible resin and a working step of coupling the pair of frame members are performed simultaneously. In addition, an integration processing can be performed using a thermal fusion bonding method, an injection molding method, a thermal compression method or an adhesive method.

As the soft and flexuous resin, for example, soft resins such as polyurethane resins and low density polyethylene can be used. Also inplace of the resin, natural or synthetic rubbers can be used.

In addition, a metal such as stainless steel or resin having a hard property such as plastic is used for the frame member. This is because hardness and strength to some extent are required for pinching the intestine with the foot part of the frame member.

It is also preferable that the cross section of the frame member is spherical, elliptical or polygonal. In order to avoid a sharp contact face so as not to injure the intestine surface, the cross section is desirably spherical or elliptical, but in order to improve a toothing upon pinching of the pair of frame members, it is also contemplated to make the frame member polygonal with or without concavoconvex portions.

In addition, as the length of the foot part of the frame member, about 30 to 70 mm is required in consideration of the length of the circumference of the intestine. When it is longer than 70 mm, a sufficient peripheral space cannot be assured in the scopic surgery to make the use difficult. To completely pinch the circumference of the intestine, it is thought that at least 30 mm is required as the length of the foot part.

It is also preferable that the accommodation bag has a translucency. For example, it is made from polyethylene as a material.

The locking part is composed of two flexible belt members and at least one or more through-holes provided in one belt member, and is constituted by disposing each belt member at substantially the center of each of the pair of frame members. Thereby, the pair of frame members is made in a closed state by inserting another belt member in the through-hole. Here, the flexible belt member refers to, for example, one formed of the soft resin and capable of being wound in a belt shape to the intestine, and includes not only the belt shape but also a string shape.

One band member is inserted from its tip end part side and passed through the through-hole in another band member. Since each band member is disposed at substantially the center of each of the pair of frame members, the pair of frame members pinches the intestine using the coupled portion as the supporting point. Different from the case of pinching with a clothepins, without forcing a working burden on the operator, it is possible to pinch the intestine with the frame members by gradually pulling the belt members while controlling and confirming the degree of constriction.

In addition, the locking part is composed of two hard bar members including the flexible belt member at one end and at least one or more through-holes provided in one belt member, and is constituted by disposing each bar member at substantially the center of each of the pair of frame members. As is the case with the above, the pair of frame members is made in the closed state by inserting another belt member in the through-hole.

Here, it is preferable to insert the tip end part of the long bar member in the through-hole to lock by providing the difference in length of two bar members; providing the through-hole in the belt member joined to the short bar member; disposing such that the position of the through-hole is fitted with the tip end part of the long bar member when the two bar members are aligned; and inserting the entire belt member joined to the long bar member from the tip end part side in the through-hole.

By making the difference in length of the two bar members and providing the through-hole in the portion located in the tip end part of the long bar member in the belt member joined to the short bar member, it becomes possible to insert the entire belt member joined to the long bar member and insert the tip end part of the long bar member in the through-hole to lock.

In addition, as the difference in length of the two bar members, the predetermined length is determined in consideration with a width of the belt member, a diameter of the bar member and a diameter of the through-hole. Considering the auxiliary tool which pinches the intestine in laparoscopic surgery, the difference in length of the two bar members is appropriately 3 to 7 mm because the width of the belt member, the diameter of the bar member and the diameter of the through-hole are several millimeters, respectively. Preferably the difference is 5 mm. In addition, by disposing the through-hole to locate in the tip end part of the long bar member when the two bar members are aligned using the coupled portion as the supporting point, it becomes possible to insert the tip end part of the long bar member in the through-hole to lock. A size of the through-hole is the size capable of inserting the belt member.

It is preferable that the bar member is the metal, or resin having a hard property and the cross section of the bar member is spherical, elliptical or polygonal. In addition, the surface of the bar member is covered with the covering member composed of the soft and flexuous resin. In addition, a tip shape of the bar member is streamlined such that the bar member is easily inserted in the through-hole and fixed, and easily removed after use.

The belt member, the covering member of the bar member and the frame member and the coupled portion are integrated using soft and flexuous resin. As this soft and flexuous resin, urethane resins and low density polyethylene can be used. Also in place of the resin, natural or synthetic rubber can be used.

In addition, the shape of the tip end part of the belt member passed through the through-hole is made thin to be easily inserted in the through-hole. It is also preferable that at least one protruding part is provided in a part of the belt member passed through the through-hole to lock the belt member so as to hardly escape after the belt member is inserted in the through-hole. By making the belt member itself have an elasticity, the belt member passed through the through-hole is made hardly escapable. In addition to these, the belt member may be locked so as to hardly escape after inserting in the through-hole by providing sawtooth like concavoconvex parts on a part of the belt member passed through the through-hole.

Also the belt member is transparent or has a color complementary to red, e.g., blue or green. This is because the belt member can be clearly recognized under a red based environment of an opening inside such as intestine and easily handled under a laparoscope. Also when the pinched state is released, the pinched state can be released by grasping the frame member and pulling one belt member having the through-hole. By making the colors of the two belt members have mutually different colors, mistakes can be prevented.

In the length of the belt members, the length of the belt member having the through-hole is shorter than the length of another belt member. By making the length of the belt member whose tip end part is passed through the through-hole longer, it becomes easier to insert in the through-hole upon operation. Also by making the lengths of the two belt members mutually different, it is possible to prevent mistakes in manipulation.

By the methods of procedures according to the stages shown in the following (1) to (5) in a surgical operation such as scopic surgery, it becomes possible to prevent any man-caused dissipation of tumor cells attributed to the intra-body cavity surgical manipulation. (1) The stage of inserting the surgical auxiliary tool for covering of an exo-serous membrane exposed tumor of the present invention from the opening portion; (2) the stage of disposing the pair of frame members in the opened state on the periphery of the area of infiltration of the serous membrane; (3) the stage of inserting the belt member in the through-hole; (4) the stage of pinching the intestine on the periphery of the area of infiltration of the serous membrane by pulling the belt member inserted in the through-hole, and covering the area of infiltration of the serous membrane with the accommodation bag; (5) the stage of exsecting the outside of the pinched intestine; and (6) the stage of taking out the surgical auxiliary tool which has pinched the intestine on the periphery of the area of infiltration of the serous membrane from the opening portion.

### [Effects of the Invention]

In the surgical auxiliary tool for covering of an exo-serous membrane exposed tumor according to the present invention, because of the above constitution, it is possible to cover the exposed tumor easily, safely and reliably even under a condition such as scopic surgery where sufficient space for use of tools cannot be assured. Therefore, by using the surgical auxiliary tool, it is possible to prevent anyman-caused dissipation of tumor cells attributed to the intra-body cavity surgical manipulation.

### [Best Mode for Carrying Out the Invention]

Embodiments of the present invention will be described in detail below with reference to the drawings. The present invention is not limited to the constitutions shown in the figures. There are optimal values for the width between the coupled portions of the frame members, the length of the foot part, the length and the diameter of the bar member depending on the site subjected to operation, and it is possible to change the designs for the shape and the dimensions of the surgical auxiliary tool according to the present invention.

### [Example 1]

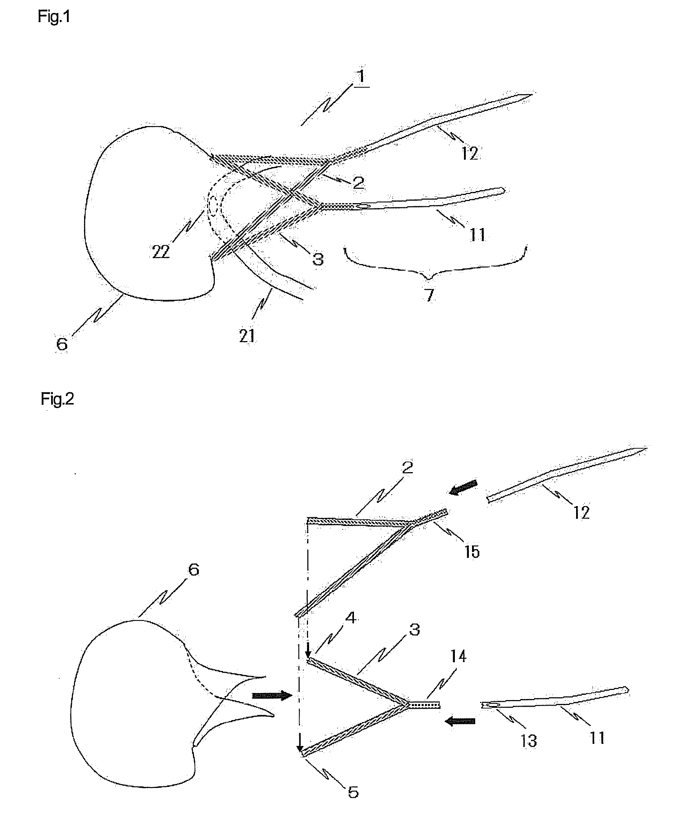

FIG. 1 shows an image figure for using one Example of the surgical auxiliary tool for covering of an exo-serous membrane exposed tumor according to the present invention. In Example 1, the entire shape of the frame member is a Y-shape. In FIG. 1, the image is shown where the periphery of the intestine (21) having the exposed tumor (22) is pinched with one pair of frame members (2, 3) with the Y-shape.

The pair of frame members (2, 3) with the Y-shape has been made from stainless steel, and its cross section shape is circular. This pair of frame members with the Y-shape is coupled at their free end portions and has a rotatably openable and closable mechanism using the coupled portion as the supporting point.

The accommodation bag (6) is a transparent polyethylene bag, and its rim of opening portion is fixed to the coupled pair of frame members (2, 3) with the Y-shape.

Further, the belt members (11, 12) formed of polyurethane resin are present on the bar members located in the center of the respective frame members (2, 3) with the Y-shape, the through-hole is provided in one belt member (11), and they constitute the locking part (7) capable of maintaining the closed state of the pair of frame members.

FIG. 2 shows an image figure for assembling the surgical auxiliary tool for covering of an exo-serous membrane exposed tumor according to Example 1. As described above, the pair of frame members (2, 3) with a Y-shape is coupled at their free end portions to form the coupled portions (4, 5), and the belt members (11, 12) which constitute the locking part are attached to the bar members (14, 15) located in the center of the frame members. In order to protect so as not to injure the intestine surface, the surface of the frame members is covered with polyurethane resin. In the actual production, three processes of covering of the frame members (2, 3), coupling of the pair of frame members (2, 3) and fabrication of the belt members (11, 12)are simultaneously processed using an integration molding method by a thermal fusion bonding method of polyurethane resin.

In addition, in order to easily attach the accommodation (6) bag to the frame members (2, 3), the shape of the rim of opening portion in the accommodation bag is processed to fit to the shape of the frame members.

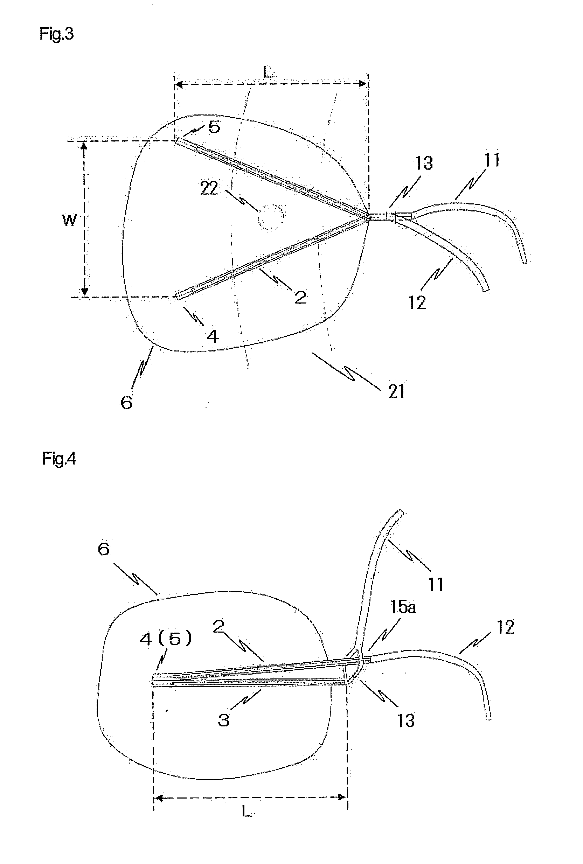

FIGS. 3 and 4 respectively show a plane view and a side view of the surgical auxiliary tool for covering of an exo-serous membrane exposed tumor according to Example 1. Here, W represents the distance between the coupled portions of the frame members and L represents the length of the foot part in the frame members. When the distance (W) between the coupled portions of the frame members is long, the intestine can be covered across a wide range. Also when the length (L) of the foot part in the frame members is long, it becomes possible to pinch the thick intestine. The designs for these parameters W and L are changed depending on the site subj ected to operation. In the case of scopic surgery, it is necessary to insert in and take out the device from the incision site, and the present surgical auxiliary tool is limited to some extent. In the case of scopic surgery, the values of the parameters W and L are empirically about 50 mm.

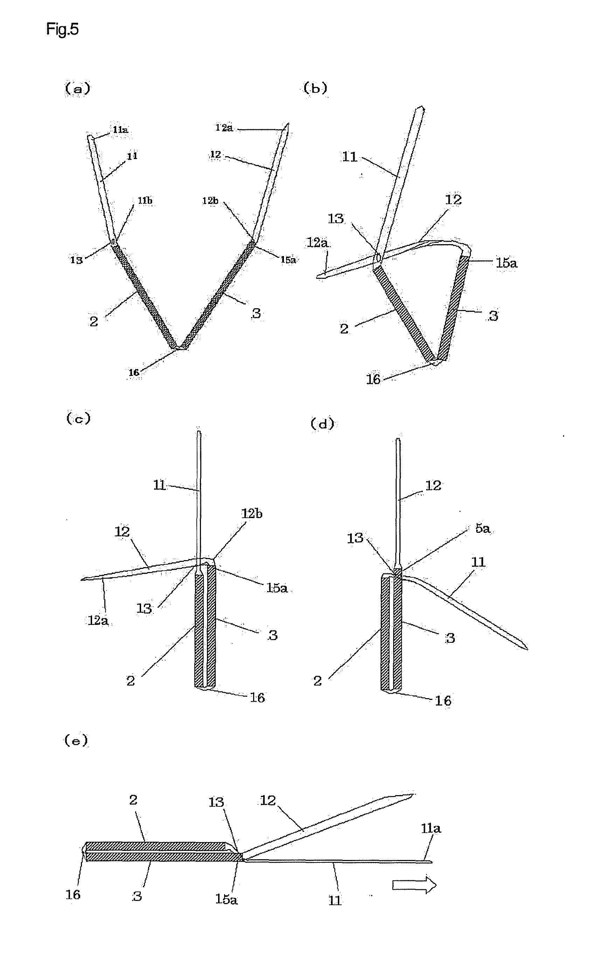

FIG. 5 shows an image figure representing how to use the locking part of the surgical auxiliary tool of Example 1. Here, FIG. 5 (a) represents the state before the belt member is inserted in the through-hole. FIG. 5 (b) represents the state where the belt member is inserted in the through-hole. FIG. 5 (c) represents the state where the entire belt member is inserted in the through-hole to pinch. FIG. 5 (d) represents the state where the tip end part of the long bar member is inserted into the through-hole to lock. These will be described below sequentially.

First, FIG. 5 (b) shows the state where the belt member (12) is inserted in the through-hole (13). By inserting one belt member (11) from its tip end part side in the through-hole (13) provided in another belt member (12) to pass through the through-hole (13), the pair of frame members (2, 3) can pinch the intestine using the coupled portion (16) as the supporting point. The belt member (12) is inserted in the through-hole (13) in the state where the periphery of the intestine is surrounded with the pair of frame members (2, 3).

Next, FIG. 5 (c) shows the state where the intestine (not shown in the figure) is pinched with the pair of frame members (2, 3) by grasping the tip end part of the belt member (11) and pulling the tip end part of the belt member (12) to pass the belt member (12) through the through-hole (13).

In addition, FIG. 5 (d) shows the state where the tip end part (15a) of the long bar member is inserted in the through-hole to lock. The difference is provided in length of the two bar members attached in the center of the pair of frame members (2,3), the through-hole (13) is provided in the belt member (11) joined to the short bar member, and the through-hole is located in the tip end part of the long bar member when the two bar members are aligned using the coupled portion (16) as the supporting point. Thus, as shown in FIG. 5 (d), by inserting the entire belt member (12) from the tip end part in the through-hole (13), the tip end part (15a) of the long bar member is inserted in the through-hole (13) to lock.

Next, the method for releasing the pinched state will be described. When the site of the intestine to be pinched is shifted or the pinched state is released, by grasping the coupled portion (16) of the frame members (2, 3) and pulling the belt member (11), the tip end part of the bar member drops off from the through-hole (13) to release the locked state.
FIG. 5 (e) shows the state where the tip end part (15a) of the long bar member is dropped off from the through-hole (13) to release the pinched state by grasping the coupled portion (16) of the frame members (2, 3) and pulling the belt member (11).

The belt member (11) is made using the soft and flexuous polyurethane resin as the material. Thus, the shape of the through-hole (13) provided in the belt member (11) deforms elliptically in a pulling direction, and it becomes easy to drop off the tip end part (15a) of the bar member from the through-hole (13). Unless the belt member (11) is pulled, the tip end part (15a) of the bar member never drops off from the through-hole (13), and they surely continue to maintain the state of pinching the intestine.

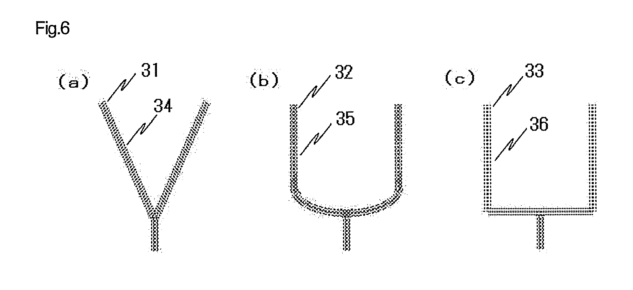

In other Examples, the entire shape of the frame member in the surgical auxiliary tool for covering of an exo-serous membrane exposed tumor according to the present invention is the U-shape or the horseshoe shape, different from the Y-shape in Example 1. FIG. 6 shows three types, i.e., the Y-shape, the U-shape and the horseshoe shape as the entire shape of the frame member. These Y-shape, U-shape and horseshoe shape have advantages that it is possible to pinch the intestine with a predetermined distance by the foot parts (34, 35, 36) toward its free end portions (31, 32, 33) as well as easily inserting and taking out the device from the incision site upon operation.
In addition, the entire shapes of the frame members shown here are some examples, and various shapes such as polygonal shapes including rectangular shapes and trapezoidal shapes, and sector forms can be conceived.

### [Industrial Applicability]

The present invention is useful as a surgical auxiliary tool for covering of an exo-serous membrane exposed tumor for the purpose of preventing dissemination and metastasis of cancer cells attributed to surgical manipulation itself in surgical operation of digestive organs and the like.

### [Brief Description of the Drawings]

FIG. 1 shows an image figure for using one Example of the surgical auxiliary tool for covering of an exo-serous membrane exposed tumor of the present invention.
FIG. 2 shows an image figure for assembly the surgical auxiliary tool for covering of an exo-serous membrane exposed tumor of Example 1.
FIG. 3 shows a plane view of the surgical auxiliary tool for covering of an exo-serous membrane exposed tumor of example 1.
FIG. 4 shows a side view of the surgical auxiliary tool for covering of an exo-serous membrane exposed tumor of example 1.
FIG. 5 shows an image figure representing how to use the locking part of the surgical auxiliary tool of Example 1. Here, FIG. 5 (a) represents the state before the belt member is inserted in the through-hole. FIG. 5 (b) represents the state where the belt member is inserted in the through-hole. FIG. 5 (c) represents the state where the entire belt member is inserted in the through-hole to pinch. FIG. 5 (d) represents the state where the tip end part of the long bar member is inserted into the through-hole to lock.
FIG. 6 (a) shows the variation of the entire shape of the frame member in the surgical auxiliary tool for covering of an exo-serous membrane exposed tumor according to the present invention: (a) Y-shape, (b) U-shape and (c) horseshoe shape.

### [Description of Symbols]

- 1.: One embodiment of the surgical auxiliary tool of the present invention
- 2.: frame member 1
- 3.: frame member 2
- 4, 5: coupled portion
- 6.: accommodation bag
- 7.: locking part
- 11.: belt member (having through-hole)
- 12.: belt member (inserted in through-hole)
- 11a, 12a: tip end part
- 11b, 12b: joined portion
- 13: through-hole
- 14: bar member (shorter)
- 15: bar member (longer)
- 15a: tip end part of bar member (longer)
- 16: coupled portion
- 21: intestine
- 22: tumor site
- 31,32,33: free end portion
- 34,35,36: foot part

## Claims

1. A surgical auxiliary tool **characterized by** comprising a pair of frame members having the respective free end portions rotatably coupled with each other; an accommodation bag having its rim of opening portion fixed to the coupled pair of frame members; and a locking part disposed at substantially the center of the frame members and capable of maintaining the closed state of the pair of frame members.

2. The surgical auxiliary tool according to claim 1 **characterized in that** an entire shape of the frame member is a Y-shape, a U-shape or more notably a horseshoe shape.

3. The surgical auxiliary tool according to claim 1 or 2 **characterized in that** a surface of the frame member is covered with a covering member composed of a soft and flexuous resin.

4. The surgical auxiliary tool according to claim 1 or 2 **characterized in that** the coupled portion of the free end portions and the covering member are integrated using a soft and flexuous resin.

5. The surgical auxiliary tool according to claim 3 or 4
**characterized in that** the soft and flexuous resin is a urethane resin or low density polyethylene.

6. The surgical auxiliary tool according to any one of claims 1 to 3 **characterized in that** the coupled portion of the free end portions has a hinge mechanism of coupling the free end portions rotatably with a pin.

7. The surgical auxiliary tool according to any one of claims 1 to 6 **characterized in that** the frame member is a metal or a resin having a hard property.

8. The surgical auxiliary tool according to any one of claims 1 to 7 **characterized in that** a cross section of the frame member is spherical, elliptical or polygonal.

9. The surgical auxiliary tool according to any one of claims 1 to 8 **characterized in that** the accommodation bag has translucency.

10. The surgical auxiliary tool according to claim 9 **characterized in that** the accommodation bag is a bag made from polyethylene.

11. The surgical auxiliary tool according to any one of claims 1 to 10 **characterized in that** the locking part is composed of two flexible belt members and at least one or more through-holes provided in one belt member, and the pair of frame members is made in the closed state by disposing each the belt member at substantially the center of each frame member and inserting another belt member in the through-hole.

12. The surgical auxiliary tool according to any one of claims 1 to 10 **characterized in that** the locking part is composed of two hard bar members comprising a flexible belt member at one end and at least one or more through-holes provided in one belt member, and the pair of frame members is made in a closed state by disposing each the bar member at substantially the center of each frame member and inserting another belt member in the through-hole.

13. The surgical auxiliary tool according to claim 12 **characterized in that** in the locking part, the two bar members are different in length, the through-hole is provided in the belt member joined to the short bar member, a position of the through-hole is disposed to fit with a tip end part of the long bar member when the two bar members are aligned, and by inserting the belt member joined to the long bar member in all parts from a tip end part into the through-hole, the tip end part of the long bar member is inserted in the through-hole to lock.

14. The surgical auxiliary tool according to claim 12 **characterized in that** the difference in length of the two bar members is 3 to 7 mm.

15. The surgical auxiliary tool according to any one of claims 11 to 14 **characterized in that** the bar member is a metal or a resin having a hard property.

16. The surgical auxiliary tool according to any one of claims 11 to 15 **characterized in that** a cross section of the bar member is spherical, elliptical or polygonal.

17. The surgical auxiliary tool according to any one of claims 11 to 16 **characterized in that** a surface of the bar member is covered with a covering member composed of a soft and flexuous resin.

18. The surgical auxiliary tool according to any one of claims 11 to 16 **characterized in that** the belt member, the covering member of the bar member and frame member and the coupled portion are integrated using the soft and flexuous resin.

19. The surgical auxiliary tool according to claim 17 or 18 **characterized in that** the soft and flexuous resin is a urethane resin or low density polyethylene.

20. The surgical auxiliary tool according to any one of claims 11 to 19 **characterized in that** the shape of the tip end part of the belt member passed through the through-hole is made thin to be easily inserted in the through-hole.

21. The surgical auxiliary tool according to any one of claims 11 to 20 **characterized in that** at least one protruding part is provided in a part of the belt member passed through the through-hole to lock the belt member so as to hardly escape after the belt member is inserted in the through-hole.

22. The surgical auxiliary tool according to any one of claims 11 to 20 **characterized in that** a sawtooth shaped concavoconvex part is provided in a part of the belt member passed through the through-hole to lock the belt member so as to hardly escape after the belt member is inserted in the through-hole.

23. The surgical auxiliary tool according to any one of claims 11 to 22 **characterized in that** the belt member has a color which is a complementary color to a red color.

24. The surgical auxiliary tool according to claim 23 **characterized in that** the belt members have mutually different colors.

25. The surgical auxiliary tool according to any one of claims 11 to 24 **characterized in that** the length of the belt member having the through-hole is shorter than the length of another belt member in the length of the belt members.

26. A method for preventing man-caused dissipation of tumor cells attributed to intra-body cavity surgical manipulation by a stage of inserting the surgical auxiliary tool according to any one of claims 11 to 25 from an opening portion; a stage of disposing a pair of frame members in an opened state on the periphery of an area of infiltration of the serous membrane; a stage of inserting a belt member in a through-hole; a stage of pinching an intestine on the periphery of an area of infiltration of the serous membrane by pulling the belt member inserted in the through-hole, and covering the area of infiltration of the serous membrane with an accommodation bag; a stage of exsecting an outside of the pinched intestine; and a stage of taking out the surgical auxiliary tool which has pinched the intestine on the periphery of the area of infiltration of the serous membrane from the opening portion, in a surgical operation such as scopic surgery.
